# EUROPEAN PATENT APPLICATION

(11) **EP 3 539 608 A1**
(43) Date of publication of application: **18.09.2019**
(21) Application number: 19162197.8
(22) Date of filing: 12.03.2019
(51) Int. Cl.: A61M 39/10, A61M 39/26

(54) **PORT CLOSURE SYSTEM**

(30) Priority: 12.03.2018 US 201862641673 P
(71) Applicant: Xerediem Medical Devices, Inc., Tucson, Arizona 85714 (US)
(72) Inventor: McEldowney, Jordan, Tucson, Arizona 85714 (US); Tomlinson, Charles, Tucson, Arizona 85714 (US)
(74) Representative: Rupprecht, Kay

(57) **Abstract**

Port closure systems and ways of using such systems are provided that include a port, a valve, and a keyway. The port establishes communication between an exterior environment and an interior volume that can receive a fluent substance. A first conduit leading to the exterior environment and a second conduit leading to the interior volume are defined by the port, where the first conduit and the second conduit follow an axis. The valve includes a flexible, resilient head extending across the port between the first conduit and the second conduit. The keyway is formed in the first conduit and defines a channel extending along the axis. The system can include a valve connector having a stem configured to be received within the port, where the stem can extend through the first conduit, the valve, and into the second conduit. The stem includes a key receivable by the keyway.

## Description

### CROSS-REFERENCE TO RELATED APPLICATIONS

This application claims the benefit of U.S. Provisional Application No. 62/641,673, filed on March 12, 2018. The entire disclosure of the above application is incorporated herein by reference.

### FIELD

The present invention relates generally to a system for accommodating the flow of a fluent substance; more particularly, to a port closure system having a flexible, resilient valve that can accommodate a valve connector, probe, or feed/drain tool for directing the flow of the fluent substance through the valve.

### INTRODUCTION

This section provides background information related to the present disclosure which is not necessarily prior art.

It would be advantageous to provide an improved system for retaining or otherwise mounting a flexible, resilient valve defining an initially closed orifice which can be opened to accommodate fluid flow through the valve, wherein the design of the system could provide advantages not heretofore contemplated in the industry or suggested by the prior art.

One type of flexible, resilient, valve is a self-closing, slit-type valve mounted in a port of a fluent container. Such valves have a slit or slits which define a normally closed orifice that opens to permit flow therethrough in response to a probe, cannula, or feed/drain tool inserted through the valve, or an increased pressure differential across the valve. Such valves are typically designed so that they automatically close to shut off flow therethrough upon removal of the probe, cannula, or feed/drain tool or a reduction of the pressure differential across the valve.

Designs of such valves and of closures using such valves are illustrated in the U.S. Pat. No. 5,271,531, No. 5,927,566, and No. 5,934,512. Typically, the closure includes a body or base mounted on the container neck to define a seat for receiving the valve and includes a retaining ring or other structure for holding the valve on the seat in the base. See, for example, U.S. Pat. No. 6,269,986 and No. 6,616,016. The valve is normally closed and can withstand the weight of the fluid product when the bottle is completely inverted so that the liquid will not leak out unless the bottle is squeezed. With such an improved system, the lid or cap need not be re-closed (although it is typically re-closed if the package is to be transported to another location, packed in a suitcase, etc.). One approach to provide a valve with hydraulic hammer resistance is shown in U.S. Pat. No. 7,784,652, titled DISPENSING VALVE WITH HYDRAULIC HAMMER RESISTANCE, the entire disclosure of which is incorporated herein by reference. Other such valve systems for use with a probe or feed/drain tool are shown in U.S. Pat. No. 8,678,249, titled VALVE MOUNTING ASSEMBLY WITH SLIT MISALIGNMENT PREVENTION FEATURE, and U.S. Pat. No. 8,316,890, titled PORT CLOSURE WITH HYDRAULIC HAMMER RESISTANCE, the entire disclosures of which are incorporated herein by reference.

While such valved systems have significant advantages and function well, it would be desirable to provide an improved system that includes multiple benefits with a minimal number of components.

### SUMMARY

The present technology includes articles of manufacture, systems, and processes that relate to controlling flow of a fluent substance, where a port closure system is provided that can be reversibly coupled to a valve connector for dispensing the fluent substance therethrough.

Various port closure systems are provided that include a port, a valve, and a keyway. The port establishes communication between an exterior environment and an interior volume that can receive a fluent substance, where the port is defined by a first conduit leading to the exterior environment and a second conduit leading to the interior volume, the first conduit and the second conduit following a first axis. The valve includes a flexible, resilient head extending across the port between the first conduit and the second conduit, the head having an exterior side facing the exterior environment, an interior side facing the interior volume, and at least one self-sealing slit formed therethrough. The keyway is formed in the first conduit, where the keyway defines a channel extending along the first axis.

The port closure system can further include the following aspects. An interior surface of the first conduit can be configured so that the interior surface does not extend laterally inwardly adjacent the valve. The interior surface of the first conduit can also provide a substantially constant diameter relative to the first axis from the exterior environment to the valve. The system can include a valve connector having a stem configured to be received within the port, where the stem extends through the first conduit, the valve, and into the second conduit when the stem is received within the port. The stem can include a key configured to be received within the keyway formed in the first conduit when the stem is received within the port. The first conduit can include a locking space configured to receive the key when the stem of the valve connector is received within the port, the key is received within the keyway, and the stem of the valve connector is rotated about the first axis. The locking space can be offset from the channel of the keyway and a protuberance can be provided between the keyway and the locking space, where rotation of the stem of the valve connector about the first axis requires the key traversing the protuberance prior to being received within the locking space. The stem of the valve connector can have an outer diameter that is larger than a diameter of a Luer fitting for an intravenous coupling. The stem of the valve connector can also have a non-tapered outer surface.

Various ways of using such port closure systems are provided that include coupling the valve connector to the port closure system, where the stem of the valve connector is received within the port, the stem extends through the first conduit, the valve, and into the second conduit, and the key is received within the keyway formed in the first conduit. The stem of the valve connector can be rotated about the first axis so that the locking space in the first conduit receives the key. Rotating the stem of the valve connector about the first axis can further include where the key traverses a protuberance between the keyway and the locking space. A fluent substance can be dispensed through the valve connector and through the port closure system. The valve connector can also be decoupled rom the port closure system.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Figure 1 is a section view showing an embodiment of a port closure system according to the present technology coupled to a tube fitment housing;
Figure 2 is an exploded isometric view of the port closure system taken from above;
Figure 3 is an exploded isometric view of the port closure system taken from below;
Figure 4 is a section of a valve of the port closure system;
Figure 5 is an isometric view of a valve connector on a distal end of a tubing prior to coupling the valve connector with the port closure system;
Figure 6 is sectional view of the valve connector coupled to the port closure system;
Figure 7 is a top plan view of the port closure system;
Figure 8 is a section view of the port closure system taken from line 8-8 in Figure 8;
Figure 9 is a section view of the port closure system taken from line 9-9 in Figure 8;
Figure 10 is an enlarged view of the area encircled by "10" in Figure 9;
Figure 11 is a side elevational view of the valve connector confronting a Luer-fitting;
Figure 12 is a sectional view of the valve connector confronting the Luer-fitting; and
Figure 13 is an enlarged view of the area encircled by "13" in Figure 12.

### DETAILED DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. Regarding methods disclosed, the order of the steps presented is exemplary in nature, and thus, the order of the steps can be different in various embodiments. "A" and "an" as used herein indicate "at least one" of the item is present; a plurality of such items may be present, when possible. Except where otherwise expressly indicated, all numerical quantities in this description are to be understood as modified by the word "about" and all geometric and spatial descriptors are to be understood as modified by the word "substantially" in describing the broadest scope of the technology. "About" when applied to numerical values indicates that the calculation or the measurement allows some slight imprecision in the value (with some approach to exactness in the value; approximately or reasonably close to the value; nearly). If, for some reason, the imprecision provided by "about" and/or "substantially" is not otherwise understood in the art with this ordinary meaning, then "about" and/or "substantially" as used herein indicates at least variations that may arise from ordinary methods of measuring or using such parameters.

All documents, including patents, patent applications, and scientific literature cited in this detailed description are incorporated herein by reference, unless otherwise expressly indicated. Where any conflict or ambiguity may exist between a document incorporated by reference and this detailed description, the present detailed description controls.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components, or process steps, the present technology also specifically includes embodiments consisting of, or consisting essentially of, such materials, components, or process steps excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

As referred to herein, disclosures of ranges are, unless specified otherwise, inclusive of endpoints and include all distinct values and further divided ranges within the entire range. Thus, for example, a range of "from A to B" or "from about A to about B" is inclusive of A and of B. Disclosure of values and ranges of values for specific parameters (such as amounts, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1-10, or 2-9, or 3-8, it is also envisioned that Parameter X may have other ranges of values including 1-9, 1-8, 1-3, 1-2, 2-10, 2-8, 2-3, 3-10, 3-9, and so on.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The present technology relates to port closure systems and ways of using port closure systems that optimize coupling to a valve connector, maintain valve effectiveness over time, and reduce or prevent misconnections with other systems. The port closure system can include a valve positioned within a port to prevent a flow of fluid therethrough (e.g. a low profile gastrostomy tube) when not connected to a valve connector, but where fluid can pass therethrough the valve when connected to the valve connector (e.g., to deliver feed/medication as well as allow for gastric decompression). The present technology can be used in a similar fashion as other systems that facilitate flow when connected and prevent backflow when disconnected, where these other systems primarily use duckbill style valves. Such duckbill valves, however, are prone to low level leakage when disconnected from their valve connectors at low pressure (e.g., stomach pressure of ≈ 1kPa). Another issue is that such duckbill valves do not completely seal at low pressures, especially after extended/repeated use due to there being no internal feature forcing the valve to completely seal and the tendency of the valves toward taking on a compression set over time - a minimum sealing pressure is required to completely prevent flow, resulting in low level leakage and necessitating a plug to be used with these devices.

The present port closure system addresses the shortcomings of such duckbill valves and provides improved performance with regards to low pressure leakage when disconnected from the valve connector. This is due in part to a valve reset feature that is effectively an interference between a portion of the port and the valve. This interference acts to force the valve to completely seal under low and high pressure environments immediately after disconnecting from the valve connector. In certain embodiments, the valve can be similar to the valve described in US Pat. No. 9,580,214 titled PORT CLOSURE SYSTEM FOR USE WITH A PROBE/FEED/DRAIN TOOL with at least one key differentiating factor, the lack of an annular flange. Within the valve described in US Pat. No. 9,580,214, there is an annular flange which is a rigid component extending over the top of the valve to hold it in place. In order to design the valve to interface with the valve connector, the flange is absent in the present technology. The valve in the present technology is unique not only in the lack of an annular flange, but also in the addition of a locking keyway within the port. This keyway interfaces with a key on the valve connector to form a secure connection between the two. This keyway can also include a locking protuberance to prevent unintended rotation/disconnection of the valve connector to the port closure system.

The valve connector provided by the present technology can interface with the port closure system to allow for fluid (e.g., liquid and/or gases) to pass through the valve. A valve connector stem opens a cross slit of the valve when inserted into the port and valve. The outer diameter of the valve connector stem provide a tight fit within the cross slit of the valve to form a seal when the valve is connected to the valve connector. The outer surface of the valve connector stem can be straight (i.e. not tapered) to provide a safety feature by preventing connection between the valve connector and Luer-tapered devices, such as intravenous lines. The safety feature can ensure that feed is not inadvertently delivered to a non-enteral route, known as a misconnection. Overall, the port closure systems provide certain benefits and advantages, including the ability to completely prevent leakage at both low and high pressure without the need for a secondary plug. Further, the valve connectors can include a stem having an outer diameter that is greater than other devices including standard Luer fittings, which builds safety into the system by preventing unintended connection with non-enteral devices that can lead to complications and issues; e.g., preventing enteral feed from being inadvertently fed into an intravenous line.

As discussed in detail hereinafter, the port closure system of the present technology can be used to provide a valve in a fluid handling system, including in an enteral tube feeding system so as to accommodate transfer of fluent substances including, but not limited to, liquid feedings carrying essential nutrients. Further, the port closure system of the present invention can be used to mount a valve in an associated container or other dispensing structure so as to accommodate transfer of fluent substances including, but not limited to, water or other fluids suitable for consumption. Notably, the port closure system is especially suitable for use with the type of flexible, resilient valve that includes a peripheral attachment portion of the valve and a central valve head, which can be openable in either of two opposite directions through one or more slits.

For ease of description, many of the figures illustrating the present technology show one form of a valve held in one embodiment of the present invention port closure system in one typical orientation that the port closure system may have in a particular application, and terms such as upper, lower, horizontal, exterior, interior, etc., are used with reference to this orientation. It will be understood, however, that the port closure system of this invention may be manufactured, stored, transported, sold, and used in orientations other than the orientation described.

The port closure system of the present invention can be used with a variety of conventional or special fluent substance handling and/or holding systems, including tube fitment housings, glass or plastic bottles, flexible tubular containment structures, containers, tanks, vessels, and other equipment or apparatus, the details of which, although not fully illustrated or described, would be apparent to those having skill in the art and an understanding of such systems. The particular fluent substance handling or holding system, per se, forms no part of, and therefore is not intended to limit, the broad aspects of the present technology.

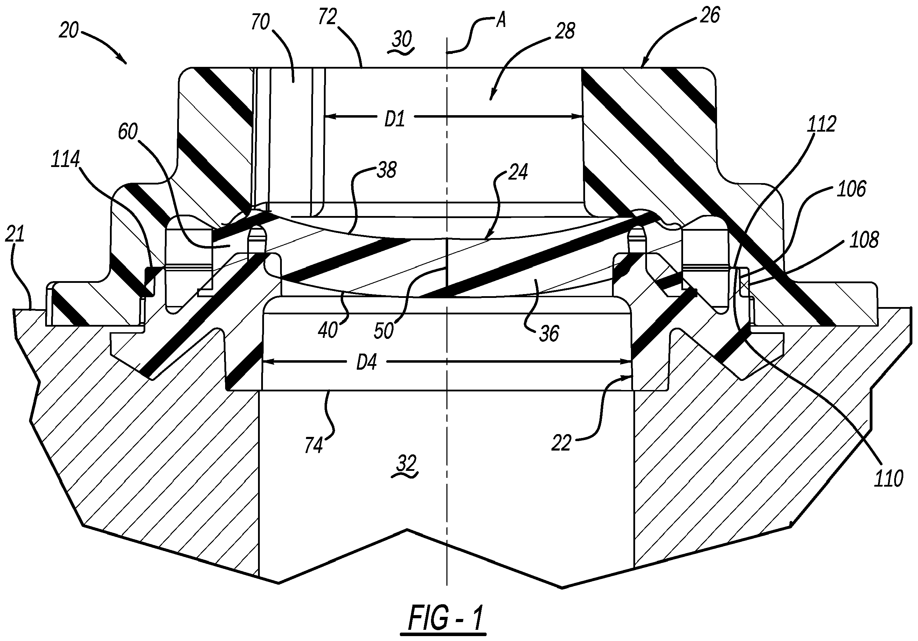

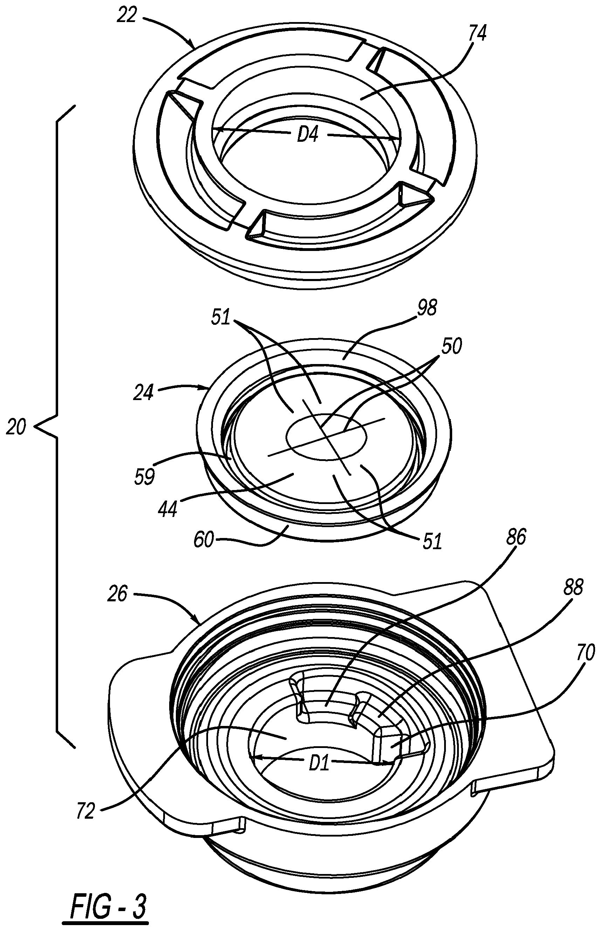

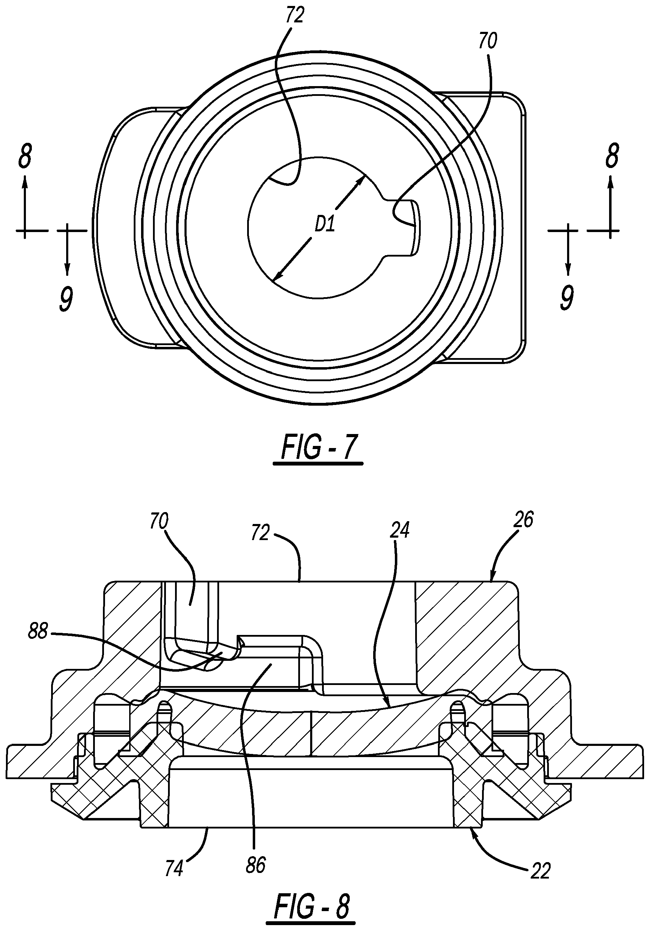

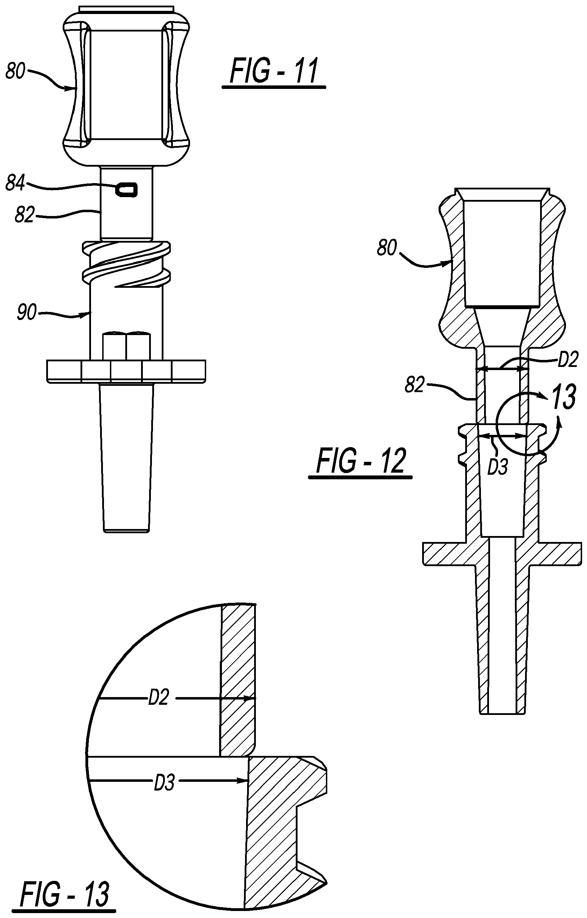

Various aspects of an embodiment of the port closure system are illustrated in FIGS. 1-13, where the port closure system is designated generally by reference numeral 20. The system 20 is illustrated in connection with a fluent system in the form of a tube fitment housing, only partially shown at 21, that can be connected to an enteral feeding tube or system. The system 20 allows the selective introduction of liquid feeding that supply critical nutrients to a patient via the enteral feeding tube or system, while maintaining a closed and lock free condition for the enteral feeding tube or system when a liquid feeding is not being provided to a patient.

The port closure system 20 includes a port 28, a valve 24, and a keyway 70. The port 28 is used to establish communication between an exterior environment 30 and an interior volume 32 that can receive a fluent substance. The port 28 is defined by a first conduit 72 leading to the exterior environment 30 and a second conduit 74 leading to the interior volume 32. As shown in the figures, the first conduit 72 and the second conduit 74 follow a first axis A. The valve 24 includes a flexible, resilient head 36 extending across the port 28 between the first conduit 72 and the second conduit 74. The head 36 has an exterior side 38 facing the exterior environment 30, an interior side 40 facing the interior volume 32, and at least one self-sealing slit 50 formed therethrough. The keyway 70 is formed in the first conduit 72, where the keyway 70 defines a channel 76 extending along the first axis A.

It can be seen that an interior surface 78 of the first conduit 72 does not extend laterally inwardly adjacent the valve 24. As such, the interior surface 78 of the first conduit 72 adjacent the valve is structurally and functionally different from the annular flange described in US Pat. No. 9,580,214, where the annular flange provided therein is a rigid component extending over the top of the valve to hold it in place. It can also be seen that the interior surface 78 of the first conduit 72 provides a substantially constant interior diameter D1 relative to the first axis A from the exterior environment 30 to the valve 24.

A valve connector 80 is provided that includes a stem 82 configured to be received within the port 28. When received within the port 28, the stem 82 extends through the first conduit 72, the valve 24, and into the second conduit 74. The stem 82 includes a key 84 configured to be received within the keyway 70 formed in the first conduit 72 when the stem 82 is received within the port 28. The first conduit 72 includes a locking space 86 configured to receive the key 84 when the stem 82 of the valve connector 80 is received within the port 28, the key 84 is received within the keyway 70, and the stem 82 of the valve connector 80 is rotated about the first axis A. As shown, the locking space 86 is offset from the channel 76 of the keyway 70. A protuberance 88 is located between the keyway 70 and the locking space 86 so that rotation of the stem 82 of the valve connector 80 about the first axis A requires the key 84 to traverse and move past the protuberance 88 prior to being received within the locking space 86.

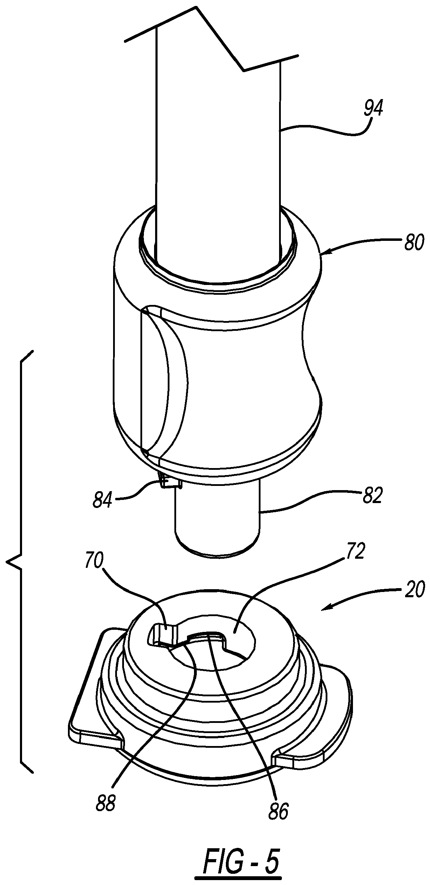

The stem 82 of the valve connector 80 has an outer diameter D2 that is larger than an inner diameter D3 of a Luer fitting 90 for an intravenous coupling. In this way, the stem 82 cannot be inserted into the Luer fitting 90 and the opportunity for a misconnection is minimized. The stem 82 of the valve connector 80 also has a non-tapered outer surface 92 that is incompatible with the Luer fitting 90, as female Luer-fittings are typically configured to receive tapered male connectors. As shown in FIGS. 5-6, a length of tubing 94 can be coupled to the valve connector 80, where the other end (not shown) can be coupled to a reservoir of a fluent substance, for example.

The embodiment of the port 28 depicted in the figures comprises a mounting fitment 26 and a retention structure 22. The mounting fitment 26 includes the first conduit 72 and the retention structure 22 includes the second conduit 74, where the valve 24 is received between the mounting fitment 26 and the retention structure 22. As shown, at least a portion of the retention structure 22 is received by the mounting fitment 26 and a portion of the valve 24 is compressed between the mounting fitment 26 and the retention structure 22. The port 28 includes a laterally inwardly facing engaging surface 34 contacting the interior side 40 of the valve 24. In the embodiment depicted in the figures, the engaging surface 34 is provided by the retention structure 22, where an interference 96 is created between the engaging surface 34 of the retention structure 22 and a laterally outwardly facing peripheral surface 54 on the interior side 40 of the valve 24. The remainder of the valve includes confronting, openable portions 51 along the self-sealing slit 50 to define a normally closed orifice in an unconstrained condition, where the openable portions 51 are operable to move in a first direction toward the interior volume 32 to an open orifice configuration and in a second direction toward the exterior environment 30 to another open orifice configuration. The laterally outwardly facing peripheral surface 54 is compressed laterally inward by engagement with the engaging surface 34 to impose a closing force on the self-sealing slit 50 to increase the resistance of the normally closed orifice to opening in at least the second direction when the valve head 36 is subjected to a pressure differential acting across the valve head 36. As such, the interference 96 between the engaging surface 34 and the peripheral surface 54 can maintain the valve 24 in the closed state, where a predefined force can be required to move the openable portions 51 along the self-sealing slit 50. The interior diameter D1 of the first conduit 72 is smaller that an interior diameter D4 of the second conduit 74, as shown. For example, the larger interior diameter D4 of the second conduit 74 can accommodate the openable portions 51 in addition to the stem 82 of the valve connector 80 when the openable portions 51 are moved in the first direction toward the interior volume 32 when the stem 82 is inserted through the valve 24.

The valve 24 is a self-closing, slit-type valve. The valve is preferably molded as a unitary structure from material which is flexible, pliable, elastic, and resilient. This can include elastomers, such as a synthetic, thermosetting polymer, including silicone rubber, such as the silicone rubber sold by Dow Corning Corp. in the United States of America under the trade designation C6-560. Another suitable silicone rubber material is sold in the United States of America under the designation Wacker 3003-60 by Wacker Silicone Company. Both of these materials have a hardness rating of 60 Shore A. The valve 24 could also be molded from other thermosetting materials or from other elastomeric materials, or from thermoplastic polymers or thermoplastic elastomers, including those based upon materials such as thermoplastic propylene, ethylene, urethane, and styrene, including their halogenated counterparts.

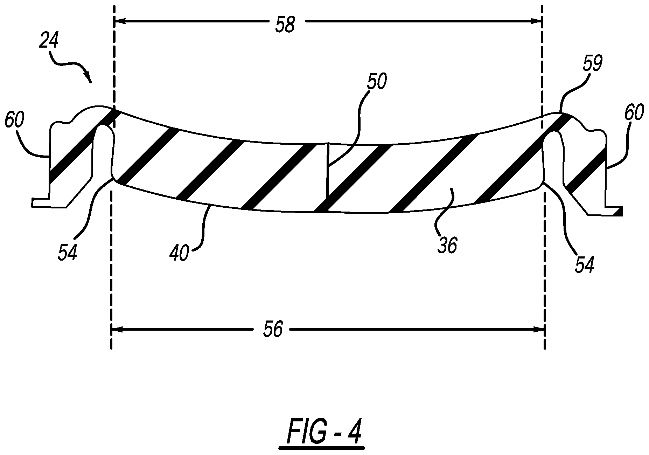

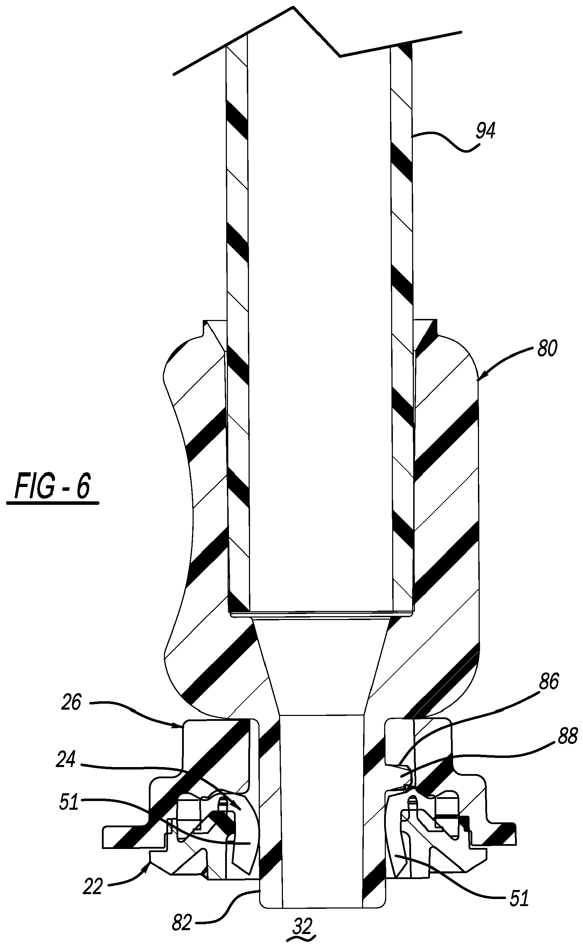

The valve 24 has a normally closed, rest position or configuration shown in FIGS. 1-4 and 8-9. The valve 24 is typically designed to remain closed when the pressure differential across the valve head 36 is below a predetermined amount. The inherent resiliency of the valve 24 allows the valve 24 to return to the normally closed condition (by action of the force generated from the resilient valve's deformational stresses). The valve 24 can be forced to one or more open positions or configurations, as shown in FIG. 6, when a sufficiently high force acts on the valve 24 as described hereinafter. As best seen in FIG. 4, the valve 24 includes a flexible, central portion or head 36 that extends laterally across the port 28, with a first or exterior side 38 and a second or interior side 40. When the valve 24 is closed, the interior side 40 has a surface that is convex and arcuate in shape, and the exterior side 38 has a surface that is concave and arcuate in shape with a planar central portion 42 and a semispherical outer portion 44. In this regard, while preferred forms of valve 24 and the head 36 are shown herein, it should be understood that other configurations, such as for example those shown in aforementioned U.S. Pat. No. 7,784,652 and U.S. Pat. No. 8,678,249 may be desirable depending upon the specific parameters and requirements of each particular application.

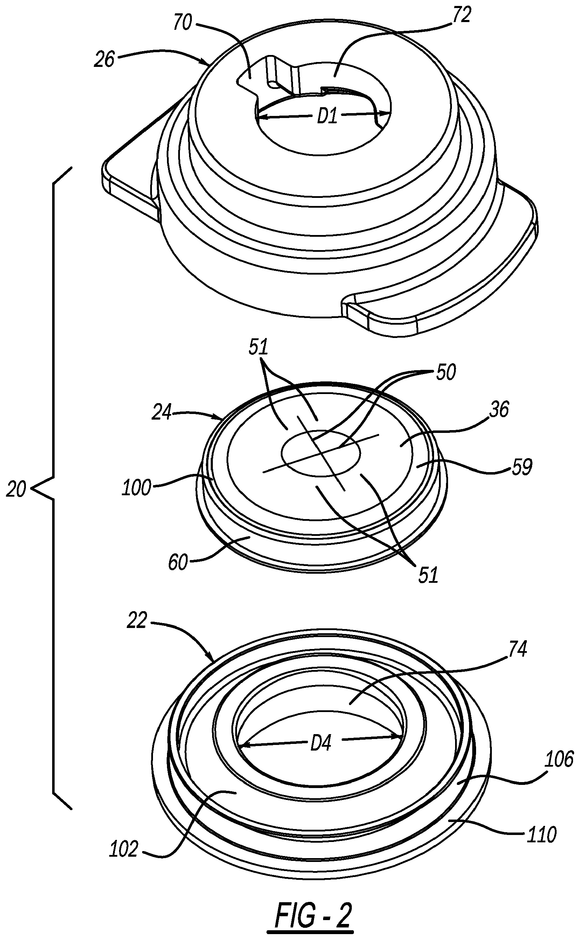

As best seen FIGS. 2-3, the head 36 has planar, intersecting, self-sealing slits 50 which together define a closed orifice when the valve 24 is closed. Preferably, the slits 50 are normal to each other and equal in length. In the illustrated forms of the valve 24, the slits 50 define four, generally sector-shaped, equally sized flaps or petals 51 in the head 36. The flaps or petals 51 may be also characterized as "openable regions" or "openable portions" of the valve head 36. Each flap or petal 51 has a pair of diverging transverse faces defined by the slits 50, and each transverse face seals against a confronting transverse face of an adjacent petal 51 when the valve 24 is closed.

The valve 24 can be molded with the slits 50. Alternatively, the valve slits 50 can be subsequently cut into the head 36 of the valve 24 by suitable conventional techniques. As another alternative, the slits 50 could be partially molded into the head 36, with the remainder being cut after molding. However the slits 50 are formed, the orifice should be closed when the valve 24 is in an unconstrained or as-molded condition. In operation, the petals 51 can be forced open inwardly in a first direction (toward the interior volume 32 in FIGS. 1 and 6) from the intersection point of the slits 50 when a sufficient force (or pressure differential) is applied to the exterior side 38 of the valve head 36, or forced open outwardly in a second direction (toward the exterior environment 30 in FIG. 1) from the intersection point of the slits 50 when a sufficient force (or pressure differential) is applied to the interior side 40 of the head 36.

FIG. 6 illustrates an example of the valve 24 opening in the first direction wherein the stem 82 of the valve connector 80 is inserted in the first direction in order to direct a liquid feed into the interior volume 32 via a flow path 53 in the stem 82. The open petals 51 accommodate the penetration of the end of the stem 82 into the interior volume 32 of the fitment 21. The petals 51 seal around the cylindrical periphery of the stem 82 in a substantially liquid-tight manner. When the stem 82 is withdrawn from the valve head 36 by movement in the second direction, the inherent resiliency of the head 36 and petals 51 return the orifice to the closed condition.

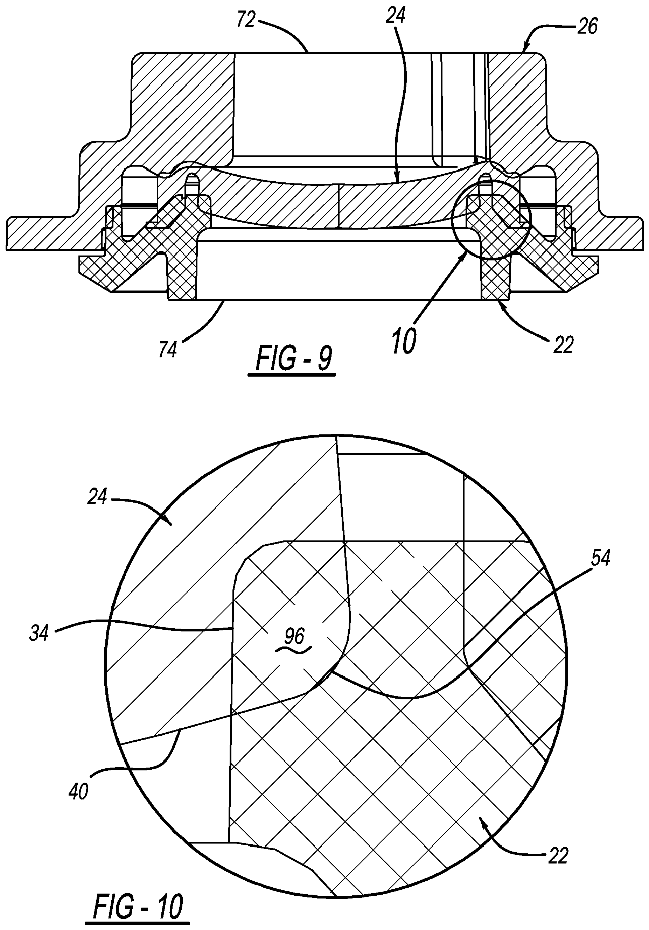

As best seen in FIGS. 2-4 and 9-10, the valve head 36 may also be characterized as having a laterally outwardly facing peripheral surface 54 at the outer periphery of the valve head 36. The surface 54 is sized and/or shaped in its as-molded or unconstrained condition so that it is compressed laterally inwardly by engagement with the surface 34 of the port 28, as best seen in FIGS. 9-10, the unconstrained size and shape of surface 54 is shown in comparison to the surface 34 on the retention structure 22. Compression of the laterally outwardly facing peripheral surface 54 of the valve 24 by the laterally inwardly facing engaging surface 34 is a result of the interference 96. This laterally inward compression of the head 36 imposes a closing force on the self-sealing slits 50 that increases the resistance of the normally closed orifice to opening in at least the second direction toward the exterior environment 30 when the valve head 36 is subjected to an increased pressure differential acting across the head 36, such as may be caused by gastric gases generated within a patient and acting through an enteral feed tube or system. It should be noted that this is particularly advantageous in connection with a system 20 wherein the valve head 36 is penetrated by the stem 82 of the valve connector 80 and must return to the closed condition and resist pressure differentials after the stem 82 is removed.

With respect to the laterally inward compression of the head 36, as best seen in FIG. 4, the surface 54 is slightly frusto-conical with a maximum diameter 56 adjacent the interior side 40 and a minimum diameter 58 adjacent a flexible, resilient intermediate portion 59 of the valve 24 that connects the head 36 to a peripheral attachment portion or flange 60 of the valve 24. The maximum diameter 56 is greater than the diameter D of the surface 34 of the retention structure 22; see FIGS 9-10. The minimum diameter 58 is preferably equal to the diameter of the surface 34, or just slightly less than the diameter. While it is believed that the illustrated shape provides a superior closing force, other shapes and/or sizes for the surface 54 that create an interference fit 96 with the surface 34 further may be desirable depending upon the particular requirements and parameters of each application.

To accommodate mounting and retention of the valve 24, the flange 60 preferably has a generally dovetail-shaped, transverse cross section which defines a pair of frusto-conical surfaces 98 and 100, as best seen in FIGS. 2-3. The retention structure 22 includes a frusto-conical surface 102 for matingly engaging the axially inwardly facing frusto-conical surface 98 of the flange 60. The mounting fitment 26 includes an axially inwardly facing frusto-conical seat 104 which is adapted to matingly engage, and clamp against, the axially outwardly facing frusto-conical surface 100 of the flange 60. The retention structure 22 further includes a radially outwardly facing cylindrical wall 106 that is received in a cylindrical opening 108 of the mounting fitment 26, with a pair of engaging shoulder surfaces 110 and 112 on the retention structure 22 and mounting fitment 26, respectively, to limit the axial engagement of the retention structure 22 into the mounting fitment 26 so as to provide the appropriate clamping force on the flange 60. Preferably, the retention structure 22 is permanently fixed or bonded within the mounting fitment 26, preferably, via ultrasonic welding in an annular zone adjacent the shoulders, as indicated at 114.

While a preferred form of mounting has been shown, the retention structure 22, valve 24 and fitment 26 could have other configurations, such as a different shape for the flange 60 and seat 80. Also, in some other arrangements, the valve 24 could be held in the mount fitment 26 by other means, such as, for example, the valve 24 could be held in by heat bonding, adhesive, and/or a press fit, etc. with or without the flange 60 and/or intermediate portion 59. As another alternative, the valve 24 could be bi-injection molded onto one of the retention structures 22 and/or the mount fitment 26.

It is to be understood that the orifice of the valve 24 may be defined by structures other than the illustrated straight slits 50. The slits may have various different shapes, sizes and/or configurations in accordance with the requirements and parameters of each particular application. For example, the orifice may also include four or more intersecting slits.

If it is desired to provide particular performance characteristics, then the system 20 is preferably configured for use in conjunction with (1) the characteristics of the particular application, which, for example, may establish the maximum anticipated pressure differential across the valve 24; (2) the characteristics of the particular substance or product to be used with the system 20; and (3) any relevant characteristics of the other components, such as the valve connector 80 and stem 82. For example, the viscosity and density of the fluent substance can be relevant factors in designing the specific configuration of the system 20 and valve 24. The rigidity and durometer of the valve material, and size and shape of the valve head 36, can also be relevant to achieving some desired characteristics, and can be selected for accommodating the normal range of pressure differential that is expected to be typically applied across the valve head 36, and for accommodating the characteristics of the substance to be used with the system 20.

It should be appreciated that the system 20 can maintain a leak-free seal between the exterior environment 30 and the interior 32 of the enteral feeding tube or system, thereby allowing for elimination of an overcap as required in some systems. In this regard, the radially inward compressive forces created by the interference 96 between the valve head 36 and the retention structure 22 serve to ensure proper closing of the valve 24 and maintaining of the valve 24 in its closed position. It should be appreciated that while the system 20 has been described herein in connection with an enteral feeding tube or system and/or in connection with a valve connector 80 including a stem 82, the system 20 may find use in other applications, connectors, tubing, dispensers, etc. and that no limitation to use with an enteral feeding tube or system and/or valve connector 80 as shown is intended unless expressly recited in the claims.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms, and that neither should be construed to limit the scope of the disclosure. In some example embodiments, well-known processes, well-known device structures, and well-known technologies are not described in detail. Equivalent changes, modifications and variations of some embodiments, materials, compositions and methods can be made within the scope of the present technology, with substantially similar results.

## Claims

1. A port closure system comprising:
a port to establish communication between an exterior environment and an interior volume that can receive a fluent substance, the port defined by a first conduit leading to the exterior environment and a second conduit leading to the interior volume, the first conduit and the second conduit following a first axis;
a valve including a flexible, resilient head extending across the port between the first conduit and the second conduit, the head having an exterior side facing the exterior environment, an interior side facing the interior volume, and at least one self-sealing slit formed therethrough; and
a keyway formed in the first conduit, the keyway defining a channel extending along the first axis.

2. The system of Claim 1, wherein an interior surface of the first conduit does not extend laterally inwardly adjacent the valve.

3. The system of Claim 1, wherein an interior surface of the first conduit provides a substantially constant diameter relative to the first axis from the exterior environment to the valve.

4. The system of Claim 1, further comprising a valve connector including a stem configured to be received within the port, the stem extending through the first conduit, the valve, and into the second conduit when the stem is received within the port, the stem including a key configured to be received within the keyway formed in the first conduit when the stem is received within the port.

5. The system of Claim 4, wherein the first conduit includes a locking space configured to receive the key when the stem of the valve connector is received within the port, the key is received within the keyway, and the stem of the valve connector is rotated about the first axis.

6. The system of Claim 5, further comprising a protuberance between the keyway and the locking space, wherein rotation of the stem of the valve connector about the first axis requires the key traversing the protuberance prior to being received within the locking space.

7. The system of Claim 4, wherein the stem of the valve connector has an outer diameter that is larger than an inner diameter of a Luer fitting for an intravenous coupling.

8. The system of Claim 4, wherein the stem of the valve connector has a non-tapered outer surface.

9. The system of Claim 4, further comprising a length of tubing coupled to the valve connector

10. The system of Claim 1, wherein the port includes a mounting fitment including the first conduit and a retention structure including the second conduit, the valve received between the mounting fitment and the retention structure.

11. The system of Claim 10, wherein at least a portion of the retention structure is received by the mounting fitment.

12. The system of Claim 10, wherein the mounting fitment and the retention structure compress a portion of the valve therebetween.

13. The system of Claim 1, wherein the port includes a laterally inwardly facing engaging surface contacting the interior side of the valve.

14. The system of Claim 13, wherein the valve includes:
confronting, openable portions along the self-sealing slit to define a normally closed orifice in an unconstrained condition, the openable portions operable to move in a first direction toward the interior volume to an open orifice configuration and in a second direction toward the exterior environment to another open orifice configuration; and
a laterally outwardly facing peripheral surface compressed laterally inwardly by engagement with the engaging surface to thereby impose a closing force on the self-sealing slit to increase the resistance of the normally closed orifice to opening in at least the second direction when the valve head is subjected to a pressure differential acting across the valve head.

15. The system of Claim 1, wherein an interior diameter of the first conduit is larger that an interior diameter of the second conduit.

16. A method of using a port closure system comprising:
providing a port closure system including:
a port to establish communication between an exterior environment and an interior volume that can receive a fluent substance, the port defined by a first conduit leading to the exterior environment and a second conduit leading to the interior volume, the first conduit and the second conduit following a first axis;
a valve including a flexible, resilient head extending across the port between the first conduit and the second conduit, the head having an exterior side facing the exterior environment, an interior side facing the interior volume, and at least one self-sealing slit formed therethrough; and
a keyway formed in the first conduit, the keyway defining a channel extending along the first axis; and
coupling a valve connector to the port closure system, the valve connector including a stem received within the port, the stem extending through the first conduit, the valve, and into the second conduit, the stem including a key received within the keyway formed in the first conduit.

17. The method of Claim 16, further comprising rotating the stem of the valve connector about the first axis so that a locking space in the first conduit receives the key.

18. The method of Claim 17, wherein rotating the stem of the valve connector about the first axis so that a locking space in the first conduit receives the key includes traversing a protuberance between the keyway and the locking space.

19. The method of Claim 16, further comprising dispensing a fluent substance through the valve connector and through the port closure system.

20. The method of Claim 19, further comprising decoupling the valve connector from the port closure system.
